# EUROPEAN PATENT SPECIFICATION

(11) **EP 0 707 487 B1**
(45) Date of publication and mention of the grant of the patent: **14.04.2004**
(21) Application number: 94918217.4
(22) Date of filing: 02.06.1994
(51) Int. Cl.: A61K 31/20, A01N 37/00, A01N 63/00, A61K 31/23, A61K 35/70, A61K 35/80, A61P 25/00, A61P 27/02

(54) **METHODS AND PHARMACEUTICAL COMPOSITIONS USEFUL FOR TREATING NEUROLOGICAL DISORDERS**
FÜR DIE BEHANDLUNG NEUROLOGISCHER ERKRANKUNGEN NÜTZLICHE METHODEN UND PHARMAZEUTISCHE ZUSAMMENSETZUNGEN
METHODE ET PREPARATIONS PHARMACEUTIQUES POUR LE TRAITEMENT DE TROUBLES NEUROLOGIQUES

(30) Priority: 09.06.1993 US 73505
(43) Date of publication of application: 24.04.1996
(73) Proprietor: Martek Biosciences Corporation, Columbia, MD 21045 (US)
(72) Inventor: KYLE, David John, Catonsville, MD 21228 (US)
(74) Representative: Cockerton, Bruce Roger
(86) International application number: PCT/US1994/006317
(87) International publication number: WO 1994/028913

(56) References cited:
- EP-A- 0 218 460
- EP-A- 0 234 733
- EP-A- 0 342 795
- EP-A- 0 347 056
- EP-A- 0 404 058
- EP-A- 0 454 102
- EP-A- 0 599 576
- EP-A- 0 622 463
- WO-A-89/00606
- WO-A-91/11918
- WO-A-92/12711
- WO-A-92/13086
- US-A- 5 130 242
- US-A- 5 198 468
- M. MARTINEZ ET AL.: "DOCOSAHEXAENOIC ACID-A NEW THERAPEUTIC APPROACH TO PEROXISOMAL-DISORDER PATIENTS" NEUROLOGY, vol. 43, no. 7, July 1993, pages 1389-1397, XP002016092
- M. MARTINEZ: "TREATMENT WITH DOCOSAHEXAENOIC ACID FAVORABLY MODIFIES THE FATTY ACID COMPOSITION OF ERYTHROCYTES IN PEROXISOMAL PATIENTS" NEW DEVELOPMENTS IN FATTY ACID OXIDATION. ED. P: COATES, vol. 375, 1992, pages 389-397, XP002068900
- MARTINEZ M: "DEVELOPMENT PROFILES OF POLYUNSATURATED FATTY ACIDS IN THE BRAIN OFNORMAL INFANTS AND PATIENTS WITH PEROXISOMAL DESEASES: SEVERE DEFICIENCY OF DOCOSAHEXAENOIC ACID IN ZELLWEGER'S AND PSEUDO-ZELLWEGER'S SYNDROMES" WORLD REVIEW OF NUTRITION AND DIETETICS, vol. 66, 1991, pages 87-102, XP000603946
- PATENT ABSTRACTS OF JAPAN vol. 014, no. 047 (C-0682), 29 January 1990 & JP 01 279827 A (TAIYO FISHERY CO LTD), 10 November 1989,
- DATABASE WPI Section Ch, Week 9019 Derwent Publications Ltd., London, GB; Class B05, AN 90-142346 XP002068901 & JP 02 086 789 A (NIPPON OILS & FATS CO LTD)
- D. BATES ET AL.: "A DOUBLE-BLIND CONTROLLED TRIAL OF LONG CHAIN N-3 POLYUNSATURATED FATTY ACIDS IN THE TREATMENT OF MULTIPLE SCLEROSIS" JOURNAL OF NEUROLOGY, NEUROSURGERY AND PSYCHIATRY, vol. 52, 1989, pages 18-22, XP002016094
- THE LANCET, Volume 339, issued 01 February 1992, A. LUCAS et al.: "Breast Milk and subsequent intelligence quotient in children born preterm", pages 261-264, especially page 263, discussion and page 264, column 1, last paragraph.
- BIOCHIMICA ET BIOPHYSICA ACTA, Volume 316, issued 1973, D.H. BEACH et al.: "Environmental Influences on the Docoshexanoate Content of the Triacylglycerols and Phophatidylcholines of a Heterotrophic Marine Dinoflagellate Crypthecodinium cohnii", pages 56-65, especially pages 63-64.
- HRUBY K.: 'Ist die Retinitis pigmentosa zweifellos unheilbar ?' MONATSBLAETTER FÜR AUGENHEILKUNDE vol. 192, no. 4, F. ENKE VERLAG STUTTGART, pages 358 - 359

## Description

### FIELD OF THE INVENTION

This invention relates to therapeutic uses for treating neurological disorders, including certain neurodegenerative diseases and psychiatric disorders, as defined in the claims by administering a composition comprising a therapeutically effective amount of an oil comprising triglycerides and/or phospholipids containing docosahexaenoic acid (DHA) and/or an oil comprising triglycerides and/or phospholipids containing arachidonic acid (ARA) to a person in need of such treatment. The oils can be administered as a pharmaceutical composition, as a dietary supplement, or in the form of a food product by replacing a portion of the vegetable oil or fat thereon.

### BACKGROUND OF THE INVENTION

The human brain and other neural tissues are highly enriched in long chain polyunsaturated fatty acids which are thought to play an important role in modulating the structure, fluidity and function of the cell membranes of these tissues. Arachidonic acid (hereafter referred to as ARA) is a long chain polyunsaturated fatty acid of the ω-6 class (5, 8, 11, 14-eicosatetraenoic acid, 20:4ω-6), and is the most abundant C₂₀ polyunsaturated fatty acid in the human body. In addition to its primary role as a structural lipid, ARA also is the direct precursor for a number of circulating eicosenoids such as prostaglandin E₂ (PGE₂) prostacyclin I₂ (PGI₂) thromboxane A₂ (TₓA₂), and leukotirenes B₄ (LTB₄) and C₄ (LTC₄). These eicosenoids exhibit regulatory effects on lipoprotein metabolism, blood rheology, vascular tone, leucocyte function and platelet activation. In humans ARA is not synthesized *de novo*, but it can be synthesized by the elongation and desaturation of linoleic acid, an essential fatty acid which must be obtained from the diet.

Docosahexanoic acid (4, 7, 10, 13, 16, 19-docosahexanoic acid 22:6ω-3) (hereinafter referred to as DHA) is the most abundant of the fatty acids of the structural components of grey matter of the human brain and other neural tissues. DHA cannot be synthesized de novo in humans, but there is some evidence that this ω-3 fatty acid can be synthesized by some cell types, such as astrocytes, if the appropriate long chain polyunsaturated fatty acids are provided in the diet. S. Moore et al., *Journ. of Neurochemistry 56* (1991) pgs. 518 to 524. Most of the DHA found in the brain and retina cell membranes is believed to be obtained from dietary sources.

The importance of providing polyunsaturated fatty acids during a period of rapid brain development to prevent irreparable damage to brain cells is well known in the art. Human infants appear to have a particularly poor ability to synthesize DHA, but any deficiencies can be compensated for by feeding infants human breast milk, which is a rich source of essential fatty acids, particularly DHA and ARA. Sanders et al., *Am. J. Clin. Nutr., 31* (1978) pgs. 805-813. Recent studies comparing the performance on standard intelligence tests of children who were fed breast milk as babies to children who were fed commercial infant formulas as babies have suggested a dose response relationship between the proportion of mother's milk in the diet and subsequent IQ. A. Lucas, R. Morley, T.J. Cole, G. Lister, and C. Leeson-Payne *Lancet 339* (1992) pgs. 261-264. These studies suggest that dietary intervention therapy can effect the levels of DHA available for structural development of the nervous system.

It has been observed that DHA levels in two major classes of phospholipid, phosphatidylethanolamine and phosphatidylcholine, are significantly reduced in the brain tissues of patients with Alzheimer's disease. Control samples taken from patients of advanced age, having no clinical manifestations of dementia or other mental disorders showed no significant changes in the fatty acid composition of these two classes of phospholipids. These results suggest that the alterations in DHA concentrations in the brain tissue of Alzheimer's patients are not the result of normal aging, but are specific for the pathological mechanisms involved in this neurodegenerative disease. M. Soderberg, C. Edlund, K. Kristensson and G. Daliner, *Lipids 26* (1991) pgs. 421-425.

Peroxisomal disorders are a group of degenerative neurological disorders characterized by increased levels of very long chain fatty acids, resulting from an impaired capacity of the effected individuals for degrading these fatty acids. These disorders are related in that they all appear to result from some defect localized in the subcellular organelles known as peroxisomes. N. Gordon "Peroxisomal Disorders", *Brain Development 9* (1987) pgs. 571-575. These peroxisomal disorders have been classified into three groups based on the extent of the loss of peroxisomal functions found in a particular disease. A. C. Theil, *European Journal of Pediatrics, 151* (1992) pgs. 117-120.

The group 1 peroxisomal disorders are characterized by a virtually complete loss of peroxisomes and peroxisomal functions. These disorders include Zellweger's syndrome, neonatal adrenoleukodystrophy, infantile Refsum disease and hyperpepecolic acidemia. The group 2 disorders are characterized by the loss of multiple peroxisomal functions and include Rhizomelic chondrodysplasia punctata and Zellweger-like syndrome. The group 3 disorders are characterized by the loss of only a single peroxisomal function and include adrenoleukodystrophy, adrenomyeloneuropathy, acyl-CoA oxidase deficiency, bifunctional protein deficiency, thiolase deficiency, hyperoxaluria type I, acatalasaemia and adult Refsum disease. Clinical presentation of patients with peroxisomal disorders shows a wide divergence in phenotypic expression which varies significantly depending upon the patient's age. However, in all patients neurological functions are progressively impaired, which often leads to deterioration of the autonomic functions and death at an early age.

Recent studies of the polyunsaturated fatty acid composition of tissues in patients with peroxisomal disorders have shown that, even though the total amount of fatty acids in these tissues was normal, there are significant changes in the fatty acid composition of the patient's tissues. These patients have a significant decrease in the total amount of DHA and ARA in their serum lipid compositions. Serum plasmalogen levels are also depressed.

Usher's syndrome is an autosomal recessive genetic disorder which is associated with the degeneration of visual cells, causing retinitis pigmentosum. The visual cells contain extremely large quantities of DHA esterified in the phospholipids of the photoreceptor membranes which make up the outer segments of the visual cells. Bazan and coworkers recently have found that the plasma phospholipids of Usher's patients contain significantly less DHA and ARA than the plasma phospholipids of unaffected individuals. N. G. Bazan, B. L. Scott, T. S. Reddy and M. Z. Pelias, *Biochem. Biophys Res. Comm. 141* (1986) pgs. 600-604.

In addition researchers have found that patients suffering from other clinical conditions, such as senile dementia, diabetes-induced neuropathy, multiple sclerosis, schizophrenia and neuropathies associated with high doses of heavy metals such as lead, aluminum, and mercury also frequently have levels of DHA and/or ARA in their serum lipids which are significantly depressed in comparison to the levels found in healthy persons. For example, recent studies have established a correlation between alterations in the levels of esterification of ARA into the phospholipids of platelets and the presence of schizoaffective disorders in patients. L. Demisch et al., *Prostaglandins Leukot. Essent. Fatty Acids 46* (1992) pgs. 47-52. Evidence of abnormal essential fatty acid biochemistry in the plasma phospholipids of patients with schizophrenia also has been reported. D. F. Horrobin, *Prostaglandins Leukot. Essent. Fatty Acids 46* (1992) pgs. 71-77.

Although researchers have made some progress in understanding neurodegenerative disorders such as Alzheimer's disease and various peroxisomal disorders, effective means of treating these disorders have remained elusive.

US-A-5,130,242 and WO89/00606 describe microbial production of unsaturated fatty acids, but do not offer any suggestions to use microbial oils in the treatment of neurologic disease.

EP-A-0404058 teaches the benefit of supplying a mixture of unsaturated fatty acids in the diets of normal infants.

EP-A-0622463 suggests obtaining DHA by culturing marine microalgae, but the only teaching of any specific use for the DHA obtained in this manner is a cross-reference to EP-A-0342795, which is discussed below.

Martinez, *et al*., in New Developments in Fatty Acid Oxidation, P. Coates ed., vol. 375, 1992, pages 389-397, and in World Review of Nutrition and Dietetics, vol. 66, 1991, pages 87-102, measured the relative amounts of various fatty acids in tissue from a patient population and a control population. Martinez observed that DHA deficiency in tissue was correlated with peroxisomal disorders, but Martinez was unable to conclude from this data what caused the DHA deficiency observed in the patient population.

JP-A-02730081 suggests obtaining ARA by culturing fungi of *Entomophthora* sp., but there is no teaching of a specific use for the ARA obtained in this manner, other than a general statement that the ARA would be available for pharmaceuticals, health foods and cosmetics.

EP-A-0342795 describes studies on learning and memory in rats fed various fatty acid derivatives. All of the diets described in the reference contain EPA and ARA in addition to DHA.

EP-A-0234733 is directed to the treatment of senility with mixtures of metabolites of gamma-linolenic acid (GLA).

Bates *et al*., in Journal of Neurology, Neurosurgery and Psychiatry, vol. 52, 1989, pages 18-22, report studies in which high EPA fish oil, which also contains a lesser amount of DHA, was administered to multiple sclerosis patients.

EP-A-0218460 is directed to the treatment of senility with mixtures of metabolites of gamma-linolenic acid (GLA).

WO92/13086 teaches methods of supplementing an individual's diet with ARA, and that such supplementation would be beneficial for individuals known to be ARA deficient

US-A-5198468, EP-A-0599576, EP-A-0454102 and EP-A-0347056 deal with the treatment of schizophrenia using fatty acid compositions.

It is an object of the present invention to provide a therapeutic use for treating neurological disorders, in which the serum, tissue or membrane levels of the essential fatty acids DHA and ARA are affected.

The present invention provides the use of an oil comprising triglycerides and/or phospholipids containing docosahexanoic acid (DHA) for the preparation of a composition for the treatment of a neurological disorder associated with a DHA deficiency-associated pathology, wherein said disorder is retinitis pigmentosum, and wherein said composition is adapted for oral, nasal or topical administration.

The present invention further provides the use of an oil comprising triglycerides and/or phospholipids containing arachidonic acid (ARA) for the preparation of a composition for the treatment of a neurological disorder associated with an ARA deficiency-associated pathology, said disorder selected from the group consisting of multiple sclerosis and retinitis pigmentosum.

The present invention further provides the use of an oil comprising triglycerides and/or phospholipids containing docosahexanoic acid (DHA) and an oil comprising triglycerides and/or phospholipids containing arachidonic acid (ARA) for the preparation of a composition for the treatment of a neurological disorder associated with a DHA and ARA deficiency-associated pathology, said disorder selected from the group consisting of multiple sclerosis and retinitis pigmentosum, wherein said composition is adapted for oral, nasal or topical administration.

The present invention further provides the use of an oil comprising triglycerides and/or phospholipids containing docosahexanoic acid (DHA), the oil having a ratio of DHA to EPA of at least 10:1, for the preparation of a composition for the treatment of a neurological disorder associated with a DHA deficiency-associated pathology, said disorder selected from the group consisting of diabetic neuropathy and multiple sclerosis.

The present invention further provides the use of an oil comprising triglycerides and/or phospholipids containing arachidonic acid (ARA), the oil having a ratio of ARA to EPA of at least 10:1, for the preparation of a composition for the treatment of a neurological disorder associated with an ARA deficiency-associated pathology, wherein said disorder is diabetic neuropathy.

The present invention further provides the use of an oil comprising triglycerides and/or phospholipids containing docosahexanoic acid (DHA), the oil having a ratio of DHA to EPA of at least 10:1, and an oil comprising triglycerides and/or phospholipids containing arachidonic acid (ARA), the oil having a ratio of ARA to EPA of at least 10:1, for the preparation of a composition for the treatment of a neurological disorder associated with a DHA and ARA deficiency-associated pathology, wherein said disorder is diabetic neuropathy.

The DHA and ARA are in the form of natural complex lipids selected from triglycerides, phospholipids and mixtures thereof. Preferably the DHA and ARA are in the form of triglycerides, although they also may be in the form of phospholipids. They are obtained as single cell microbial oils by the cultivation of DHA-producing microorganisms or ARA-producing organisms under oil-producing conditions.

According to preferred embodiments of the present invention, microorganisms capable of producing a single cell microbial oil containing DHA or ARA are cultivated in a fermentor in a nutrient solution capable of supporting the growth of such organisms. Preferably the microbial oil produced is enriched in the fatty acids of interest, meaning that it will contain at least about 20% DHA or 10% ARA by weight.

Any microorganism capable of producing a microbial oil containing DHA or ARA can be used in the present invention. These microorganisms can be identified by determining whether DHA or ARA oils are present in the fatty acid profiles of the harvested biomass from a culture of the microorganism. These profiles are typically obtained by gas chromatography of methyl ester derivatives of the fatty acids present in a sample.

As used herein, the term microorganism, or any specific type of microorganism, includes wild type strains, mutant strains or recombinant strains. Wild-type and recombinant microorganisms designed to produce microbial cell oil containing DHA or ARA can be used to produce the DHA-containing and ARA-containing microbial oils. Such recombinant strains would include those designed to produce greater quantities of DHA or ARA in the single cell oil, greater quantities of total oil, or both, as compared to the quantities produced by the same wild type microorganism, when provided with the same substrates. Microorganisms selected or designed to efficiently use more cost-effective substrates, while producing the same amount of single cell oil containing DHA or ARA as the wild-type microorganism, are particularly useful for preferred embodiments of the present invention.

For the production of DHA-containing microbial oils, species of photosynthetic algae such as *Chattonella, Skeletonema, Thalassiosira, Isochrysis, Hymenomonas*, or *Cryptomonas* can be used. Preferred microorganisms are heterotrophic species of algae which include, but are not limited to, the *Dinophyceae*, for example, *Crypthecodinium*; or to fungi such as *Chytridiomycetes*, for example, *Thraustochytrium*, or *Schizochytrium* or to *the Oomycetes*, for example, *Mortierella, Saprolegnia or Mucor*.

Preferred microorganisms for producing DHA are dinoflagellates, including *Crypthecodinium*. Especially preferred is *Crypthecodinium cohnii*, an obligate heterotroph, which is described in U.S. Application Serial No. 479,135, filed February 13, 1990. *C. cohnii* is preferred because it produces fatty acids in which DHA is the only polyunsaturated fatty acid present in quantities greater than about 1% of the total amount of polyunsaturated fatty acids, a quantity which is significant for carrying out the methods of the present invention. Samples of one strain of *C. cohnii*, which produces abundant levels of DHA, have been deposited with the American Type Culture Collection at Rockville, Maryland, and assigned ATCC accession number 40750.

Microorganisms useful for producing ARA include species of algae such as *Porphyridium, Ochromonas* and *Euglena*, and fungi such as *Pythium* and *Mortierella*. Many of those species which make ARA also produce significant quantities of eicosapentaenoic acid (EPA) in addition. Unexpectedly, it has been found that *P. insidiosum and M. alpina* produce ARA but are at least substantially free of EPA. "Substantially free" is defined to mean the ratio of ARA to EPA is at least 5:1. Preferably, the ratio is at least 10:1. Most desirably, no more than 1% of the fatty acid content of the oil is EPA. As with fish oils, high EPA levels in dietary supplements result in a depression of the ability to form ARA from dietary linoleic acid. Furthermore, the administration of EPA-containing fish oils to patients, especially elderly, hypertensive or pregnant patients who may have increased prothrombin times, is undesirable because of the blood thinning effects of EPA. Accordingly, while those fungal species producing both ARA and EPA can be utilized in the process of this invention, it is preferable to use species which do not produce significant quantities of EPA. Preferred species include *Pythium insidiosum* and *Mortierella alpina*. Both species are available commercially and strains are on deposit with the American Type Culture Collective in Rockville, Maryland, such as those having ATCC accession numbers 28251 and 42430, respectively.

Likewise, although microbial species producing both DHA and EPA can be utilized as a source of the DHA oil used in this invention, it is preferable to use species which are at least substantially free of EPA. Preferably, the ratio is at least 10:1. Most desirably, no more than 1% of the fatty acid content of the oil is EPA.

### Production of DHA-Containing Oil

The DHA-producing microorganisms can be cultivated in a simple medium containing a carbon source such as glucose and a nitrogen source such as yeast extract or peptone. Use of these components in a solution such as seawater provides economically significant growth rates and cell densities. During the course of a 3 to 5 day fermentation, for example, *C. cohnii* cell densities of at least 10 grams of biomass per liter of solution, and preferably from 20 to about 40 grams per liter, can be attained.

Although cultivation can occur in any suitable fermentor, preferably the organism is grown either in a stirred tank fermentor or in an air lift fermentor. When a stirred tank fermentor is selected, agitation is provided using either Rushton-type high efficiency turbines or pitched-blade or marine impellers. Agitation and sparging renew the supply of oxygen to the microorganisms. The rate of agitation normally is increased as the biomass increases, due to the increased demand for oxygen. It is desirable to keep the tip speed at not greater than about 500 cm per sec, preferably not greater than about 300 cm per sec. Selection of strains of microorganisms which are capable of withstanding greater tip speeds without undergoing shear damage is within the purview of those of skill in the art.

The organisms used for the production of DHA-containing oil can be grown in any suitable nutrient solution. As noted above, seawater is an acceptable medium for the nutrient solution for many organisms. The seawater can be either natural, filtered or an artificial mix, each of which can be diluted with water to reduced salinities, such as 1/2 to 1/4 normal strength, or concentrated to 2 times normal strength. A preferred medium is Instant Ocean® brand artificial seawater, or alternatively a mixture of 4.5 to 20 g per liter NaCl, 1.23 g per liter MgSO₄^{·}7H₂O and 90 mg per liter CaCl₂ in water. Micronutrients can be added and may be required when using defined media. However, such micronutrients are known to those of skill in the art and generally are present in seawater or tap water. If the organism selected is heterotrophic, such as *Crypthecodinium* and *Thraustochytrium*, then a reduced carbon source is added. *Crypthecodinium* and *Thraustocytrium* require a reduced carbon source for growth.

Preferably, after addition of the seawater medium to the fermentor, the fermentor containing the medium is sterilized and cooled prior to adding the nutrients and a seed culture of the microorganism to be cultivated. Although it is acceptable to sterilize the nutrients together with the seawater, sterilization in this manner can result in a loss of available glucose. The nutrients and microorganism can be added simultaneously or sequentially.

An effective seed concentration can be determined by those of skill in the art. When a stirred tank fermentor is used, the addition of a population of from about 0.05 to 1.0 grams of dry weight equivalent per liter at the beginning of the fermentation is preferred. For example, at least about 1 to 5 x 10⁶ cells of *C. cohnii* per ml would be suitable. Thus, for a 30 liter fermentor, 1 to 3 liters of seeding media, containing viable cells at a density of 10 to 20 grams dry weight per liter would be added.

Oxygen levels preferably are maintained at a dissolved oxygen of at least about 10% of air saturation level. Biosynthesis of DHA requires oxygen and, accordingly, higher yields of DHA require dissolved oxygen levels at from about 10% to 50% of air saturation levels. For example, agitation tip speeds of 150 to 200 cm per sec in combination with an aeration rate of 1 volume of air per volume of fermentor per minute (VVM) provides dissolved oxygen levels of from about 20% to about 30% at biomass densities of about 25 grams dry weight per liter of culture for *C. cohnii*. Higher cell densities may require higher dissolved oxygen levels, which can be attained by increased aeration rates by O₂ sparging or by increasing the air pressure in the fermentor.

Acceptable carbon sources are known to those of skill in the art. For example, carbon can be provided to in the form of mono or di-saccharides such as sucrose, ldactose, fructose or glucose. Autotrophs utilize carbon dioxide as a carbon source. Many organisms also will grow on other reduced, more complex, carbon sources, such as molasses, high fructose corn syrup and hyolyzed starch. Typically, a fermentation is initiated with about 20 to 50 grams per liter glucose. More glucose is added during the fermentation as required. Alternatively, from about 50 to 150 grams glucose per liter initially can be added, thereby minimizing the frequency of future additions. The amount of carbon source provided to other organisms can readily be determined by those of skill in the art.

In addition to a reduced carbon source, a nitrogen source, such as yeast extract or peptone, is provided to the medium. For example, Difco or Marcor brand yeast extract and Sheftone brand peptone can be used. Yeast extract and peptone are organic nitrogen sources which also contain micronutrients. Other nitrogen sources easily can be determined by those of skill in the art. However, such compounds are generally more expensive than yeast extract. Any DHA- or ARA-producing algae strain variant capable of using urea, ammonia or nitrates as a nitrogen source can be used in this invention.

Typically, the fermentation is initiated with about 6 to 12 grams yeast extract per liter. More yeast extract can be added as required. A typical fermentation run requires from about 8 to 15 grams yeast extract per liter over the course of the run. Accordingly, that amount of yeast extract can be added initially with a reduced need for further additions. The precise amount can be determined by those of skill in the art. Generally, the ratio of glucose to yeast extract is from about 2:1 to about 25:1.

Cultivation can be carried out at any life-sustaining temperature. Generally, microorganisms such as *Crypthecodinium or Thraustochytrium* will grow at temperatures ranging from about 15°C to 34°C. Some fungi grow effectively at temperatures ranging from about 10°C to 80°C. Preferably, the temperature is maintained at about 20°C to 30°C. Strains which grow at higher temperatures are preferred, because they have a faster doubling time, thereby reducing total the fermentation time. Appropriate temperature ranges for other microorganisms are readily determined by those of skill in the art.

Cultivation can be carried out over a broad pH range, typically from about pH 5.0 to 9.0. Preferably, a pH range of from about 6.0 to about 7.0 is used for the growth phase. A base, such as KOH or NaOH, is used to adjust the media pH prior to inoculation. During the later stages of the fermentation, the pH of the culture medium can increase or decrease as nutrients are utilized. If desired, the pH can be adjusted during the fermentation to correct alkalinity or acidity during the growth phase by adding an appropriate acid or base.

Production of the microbial cell oil is induced in the microorganisms by the induction of a stationary phase by allowing the culture to reach a phase of nitrogen depletion or phosphate depletion or by allowing the pH of the culture to rise. Yeast extract deficiencies can be caused by providing only a limited amount of yeast extract such that the medium is depleted of its nitrogen source, while available glucose levels remain adequate for growth. It is the carbon source to nitrogen source ratio which promotes the efficient production of the single cell oil. Using glucose and yeast extract as examples, a preferred ratio of carbon source to nitrogen source at the time of inoculation is about 10 to 15 parts glucose to 1 part yeast extract. Similar ratios for other carbon and nitrogen sources can be calculated by those of skill in the art.

After induction of oil production, the culture is grown for about 24 additional hours. During this period the single cell oil containing DHA is being synthesized and oil droplets are visible inside the cells when they are observed using a microscope. Those of skill in the art can readily calculate the time of fermentation required to achieve the expected amount of cell biomass based upon the added amount of yeast extract. When that time has passed, the culture is grown for an additional 24 hours and harvested. In general, for example, the *Crypthecodinium* or *Thraustochytrium* cells are cultivated for about 60 to about 90 hours, although this time is subject to variation.

Using the *Crypthecodinium* strain designated as ATCC accession number 40750, as an example, from about 15 to 30% of the resulting biomass comprises extractable oil- Strain selection can increase this percentage. Preferably, the oil comprises greater than about 70% triglycerides having, in general, the following fatty acid composition.

| | |
|---|---|
| 15-20% | myristic acid (C_{14:0}) |
| 15-25% | palmitic acid (C_{16:0}) |
| 5-15% | oleic acid (C_{18:1}) |
| 30-50% | DHA (C_{22:6}) |

The crude oil is characterized by a yellow-orange color and is liquid at room temperature. Desirably, the oil contains at least about 20% DHA by weight, preferably about 40% DHA by weight, and most preferably at least about 50% DHA by weight.

The organisms are harvested by conventional means, known to those of skill in the art, such as centrifugation, flocculation or filtration, and can be processed immediately or dried for future processing. In either event, the oil can be extracted readily with an effective amount of solvent. Suitable solvents can be determined by those of skill in the art. However, preferred solvents include pure hexane and supercritical fluids, such as supercritical CO₂. Certain lipophilic antioxidants such as β-carotene, α-tocopherol, ascorbyl palmitate, and BHT can be added prior to extraction. These compounds help protect the oil from oxidation during the extraction and refining processes.

General extraction techniques using supercritical fluids have been developed for the extraction of oil from oil-rich plant seeds (McHugh et al. Supercritical fluid Extraction, Butterworth, 1986). However, these standard methods generally are not applicable to the extraction of microorganisms. For example, spray dried algal cells have the consistency of flour, and the flow of supercritical CO₂ is restricted as the microorganism biomass is compressed. In addition, the cell walls of microalgae and fungi are chemically dissimilar to those of most seed oil material. In order to prevent the compression and allow efficient flow and extraction, the algal biomass can be mixed with from 0.1 to 5.0 parts of lipid free structural agent, such as Celite, or diatomaceous earth. In a 50 ml reaction vessel at 450 Atm. and 100°C., 86% of the oil was extracted from C. cohnii in 25 minutes, and 100% was extracted in 85 minutes.

If the extraction solvent is hexane, a suitable ratio of hexane to dry biomass is about 4 liters of hexane per kilogram of dry biomass. The hexane preferably is mixed with the biomass in a stirred reaction vessel at a temperature of about 20 to 50°C for about 2 hours. After mixing, the biomass is filtered and separated from the hexane containing the oil. Alternatively, a wet biomass paste that is from 30 to 35% solids can be extracted directly with more polar solvents, such as ethanol, isopropanol or mixtures of hexane and isopropanol.

The solvent is removed from the oil by distillation techniques known to those of skill in the art. Conventicnal seed oil processing equipment is suitable to perform the filtration, separation and distillation. Additional processing steps, known to those of skill in the art, can be performed if required or desirable for a particular application. These steps also will be similar to those involved in conventional vegetable oil processing and allow the separation of DHA-enriched polar lipid fractions.

### ARA-Containing Oil Production

ARA producing fungi or algae are cultivated under suitable ARA-containing oil-producing cultivating conditions. If desired, the microorganism can be grown in a shake flask initially and then transferred to a fermentor. The composition of the growth medium can vary but always contains carbon and nitrogen sources. A preferred carbon source is glucose, amounts of which can range from about 10 to 200 grams glucose per liter of growth medium. Typically about 50 grams per liter are utilized for shaker flask culture. The amount can be varied depending upon the desired density of the final culture. Other carbon sources which can be used include molasses, high fructose corn syrup, hydrolyzed starch or any other low cost conventional carbon source used in fermentation processes. Additionally, lactose can be provided as a carbon source. Thus, whey permeate, which is high in lactose and is a very low cost carbon source, can be used as a substrate. Suitable amounts of these carbon sources can readily be determined by those of skill in the art. Usually, additional amounts of the carbon source needs to be added during the course of the fermentation.

Nitrogen typically is provided in the form of yeast extract at a concentration of from about 2 to about 15 grams per liter of growth medium. Preferably, about 8 to 10 grams per liter are provided. Other nitrogen sources can be used, including peptone, tryptone, corn steep liquor, etc. The amount to be added of these sources can easily be determined by those of skill in the art. Nitrogen can be added throughout the cultivation or in a batch mode, i.e. all at one time prior to cultivation.

After cultivation for 3 to 4 days at a suitable temperature, typically about 25 to 30°C, an amount of fungi or algae has grown which is sufficient for use as an inoculum in a conventional stirred tank fermentor or an air lift fermentor. Fermentation can be carried out in batch, fed-batch, or continuous fermentation modes. The stirred tank fermentor is equipped with either a Rushton-type turbine impeller or, preferably, a marine-type axial impeller.

The fermentor is prepared by adding the desired carbon and nitrogen sources. For example, a 1.5 liter fermentor can be prepared by mixing about 50 grams of glucose and about 6 grams of yeast extract per liter of water. As previously discussed, other carbon or nitrogen sources or mixtures thereof can be used.

The reactor containing the nutrient solution should be sterilized by, for example, heating prior to inoculation as described above in the discussion of microorganism cultivation for the production of DHA. After cooling to about 30°C, the inoculum can be added, and cultivation initiated. Gas exchange is provided by air sparging. The air sparging rate can vary, but preferably is adjusted to from about 0.5 to about 2.0 volumes of air per volume of fermentor per minute. Preferably the dissolved oxygen level is kept at from about 10% to about 50% of the air saturation value of the solution. Accordingly, adjustments in the sparging rate may be required during cultivation.

Agitation is desirable during fermentation. The agitation is provided by the impeller. Agitation tip speed preferably is set within the range of from about 50 cm per sec to about 500 cm per sec, preferably from about 100 to 200 cm per sec.

In general, the amount of inoculum used in a fermentation can vary. Typically, a logarithmically growing culture that is from about 2% to about 10% of the total volume of the medium in the fermentor can be used as an inoculum.

Nutrient levels should be monitored. When glucose levels drop below 5 grams per 1, additional glucose should be added. A typical cultivation cycle utilizes about 100 grams of glucose and about 15 grams of yeast extract per liter. It is desirable to deplete the nitrogen during the course of the cultivation as this enhances oil production by the fungi or algae. This is especially true when *M. alpina* is used as the production organism.

Occasionally, the culture will produce an excessive quantity of foam. Optionally, an antifoaming agent, such as those known to those of skill in the art, for example Mazu 310®, can be added to prevent foaming.

The temperature of cultivation can vary. However, those microorganisms which produce both ARA and EPA tend to produce less EPA and more ARA when cultivated at higher temperatures. For example, when *Mortierella alpina* is cultivated at less than 18°C, it begins to produce EPA. Thus, it is preferable to maintain the temperature at a level which induces the preferential production of ARA. Suitable temperatures are typically from about 25°C to about 30°C.

Preferably, cultivation continues until a desired biomass density is achieved. A desirable biomass is about 15-40 grams per liter of the organism. Such a biomass typically is attained within 48 to 72 hours after inoculation. At this time, the organisms typically contain about 5 to 40% complex lipids, of which about 10 to 50% is ARA, and can be harvested.

Harvesting can be done by any suitable method such as filtration, centrifugation, or flocculation. Because of lower cost, filtration may be preferred.

After harvesting, the biomass can be extracted without drying. Optionally, the biomass can have any residual water removed, as by vacuum drying, fluid-bed drying or lyophilization, prior to extraction. If the water is removed, it is preferable to use nonpolar solvents to extract the ARA-containing oil. While any non-polar extract is suitable, hexane is preferred. Supercritical fluids such as CO₂ or NO, as discussed above also can be used for extraction of ARA-enriched oils from algae and fungi. Although fungi such as *M*. *alpina* are unexpectedly difficult to extract with CO₂, as much as 89% of the oil of a fungal biomass can be recovered at temperatures above 90°C and pressures of 400 Atm. Alternatively, the wet biomass, which typically contains about 30 to 50% solids, can be crumbled and extracted directly using polar solvents such as ethanol, isopropyl alcohol or a mixture of hexane and isopropyl alcohol.

A preferable method of aqueous extraction involves mixing the biomass with the polar solvent isopropyl alcohol in a suitable reaction kettle. Such kettles are known. The use of three to six parts of solvent per part of biomass is desired. Most preferably, the mixing is done under nitrogen or with the addition of anti-oxidants such as β-carotene, α-tocopherol, ascorbyl palmitate or BHT to prevent the oxidation of the ARA in the lipid extract.

The solvent is removed from the oil as discussed in the section above regarding the production of a DHA-containing oil. Additional steps to further purify the oil also can be performed.

Yields can vary from about 5-50 grams of ARA-containing oil per 100 grams of dried biomass. In the case of *M. alpina*, 10 to 50 grams of triglyceride per 100 grams of dry biomass can be obtained. In the case of *Ochromonas*, 5 to 20 grams of triglyceride per 100 grams of biomass can be obtained.

Preferably the oil from *M. alpina* comprises greater than about 70% triglycerides having, in general, the following fatty acid composition:

| | |
|---|---|
| 5 - 15% | palmitic acid |
| 15 - 20% | stearic acid |
| 5 - 10% | oleic acid |
| 6 - 10% | linoleic acid |
| 2 - 10% | linolenic acid |
| 2 - 10% | dihomo-gamma linolenic acid |
| 40 - 50% | arachidonic acid |

### Administration of DHA and ARA Containing Oils

In accordance with this invention, DHA-containing microbial oils, ARA-containing microbial oils or suitable combinations of these oils are administered to patients affected by a neurological disorder characterized by depressed levels of DHA and/or ARA in the blood or tissues in comparison to the levels found in healthy individuals. The specific course of treatment administered will be determined based on normalization of serum and erythrocyte DHA and ARA levels. These serum levels of DHA and ARA are thought to reflect the long chain polyunsaturated fatty acid compositions of neurological membranes. In some cases, serum levels of ARA and DHA may need to be increased to 4 to 5 times the levels which are considered to be normal in the general population in order to see a therapeutic effect. Patients suffering from disorders involving such conditions as retinitis pigmentosum or senile dementia may respond to the administration of DHA-containing oil alone, while patients suffering from conditions such as adrenoleukodystrophy, diabetes-induced neuropathy or schizophrenia may respond more favorably to the administration of a combination of a DHA-containing oil and an ARA-containing oil. Still other patients may benefit from the administration of an ARA containing microbial oil alone.

The course of treatment can be followed by measuring levels of the fatty acid(s) of interest in the serum of treated patients. For some patients it will be possible to follow the normalization of DHA or ARA levels in neural tissue by measuring the levels of DHA and ARA in erythrocytes or in serum lipids during treatment.

Although the DHA- and/or ARA-containing oils can be administered to patients directly, more commonly they will be combined with one or more pharmaceutically acceptable carriers and, optionally, other therapeutic ingredients. Acceptable carriers are carriers which are compatible with the other components of the formulation and not deleterious to the patient.

Formulations include those suitable for oral, nasal, topical or parenteral (including subcutaneous, intramuscular, intravenous and intradermal) administration. It will be appreciated that the preferred formulation can vary with the condition and age of the patient. The formulations conveniently can be presented in unit dosage form, e.g., emulsions, tablets, and sustained release capsules, and can be prepared by any suitable pharmaceutical method.

Formulations of the present invention suitable for oral administration can be presented as discrete units such as capsules or tablets, each of which contains a predetermined amount of DHA or ARA oil or a predetermined amount of a suitable combination of DHA and ARA oils. These oral formulations also can comprise a solution or a suspension in an aqueous liquid or a non-aqueous liquid. The solution can be an emulsion such as an oil-in-water liquid emulsion or a water-in-oil liquid emulsion. The oils can be administered by adding the purified and sterilized liquids to a prepared enteral formula which is then placed in the feeding tube of a patient who is unable to swallow.

In one preferred embodiment, the DHA or ARA microbial oil is incorporated into gel capsules such as those described in Example 6. However, it will be recognized that any known means of producing gel capsules can be used in accordance with the present invention.

Compressed tablets can be prepared by compressing or molding the microbial oil(s) in a suitable machine. The oil(s) can be mixed with dry inert accessory ingredients such as carboxymethyl cellulose. The tablets optionally can be coated or scored and can be formulated so as to provide slow or controlled release of the active ingredient therein.

Other formulations suitable for topical administration include lozenges comprising DHA oil, ARA oil or a combination thereof in a flavored basis, usually sucrose and acacia or tragacanth.

Formulations suitable for topical administration to the skin can be presented as ointments, creams and gels comprising the DHA and/or ARA oil(s) in a pharmaceutically acceptable carrier. A preferred topical delivery system is a transdermal patch containing the oil to be administered.

In formulations suitable for nasal administration, the carrier is a liquid, such as those used in a conventional nasal spray or nasal drops.

Formulations suitable for parenteral administration include aqueous and non-aqueous sterile injection solutions which optionally can contain anti-oxidants, buffers, bacteriostats and solutes which render the formulation isotonic with the blood of the intended recipient; and aqueous and non-aqueous sterile suspensions which can include suspending agents and thickening agents. The formulations can be presented in unit-dose or multi-dose containers. A preferred embodiment of the present invention includes incorporation of the DHA and/or ARA oil(s) into a formulation for providing parenteral nutrition to a patient.

The microbial oil compositions of the present invention need not be administered as a pharmaceutical composition. They also can be formulated as a dietary supplement, such as a vitamin capsule or as food replacement in the normal diet. The microbial oils can be administered as a cooking oil replacement formulated so that in normal usage the patient would receive amounts of DHA and/or ARA sufficient to elevate the concentrations of these fatty acids in the serum and in membranes of affected neural tissues to normal or near-normal levels. A special emulsion type margarine could also be formulated to replace butter or ordinary margarine in the diet. The single cell microbial oils could be added to processed foods to provide an improved source of ω-3 and ω-6 unsaturated fatty acids. The oil can be microencapsulated using gelatin, casein, or other suitable proteins using methods known in the art, thereby providing a dry ingredient form of the oil for food processing. Such methods of administration can be preferred in the case of a person known to have a genetic predisposition to a disorder associated with a DHA or ARA metabolic deficiency such as a neurodegenerative disease, for example Huntington's disease or Alzheimer's disease. Providing such an individual with a dietary replacement can provide a significant prophylactic effect, delaying the onset of symptoms of a particular disorder. The administration of the long chain polyunsaturated fatty acids DHA and ARA offer a significant advantage over merely obtaining linoleic and linolenic acid, the precursors of these fatty acids, from standard foods or specialty oils such as primrose or borage oil. The administered DHA and ARA are already present in their active forms so that the patient is not required to metabolize dietary precursors. This results in the effective doses which are significantly lower than those of the precursors which would be required to produce the therapeutic effect.

It should be understood that in addition to the ingredients particularly mentioned above, the formulations of this invention can include other suitable agents such as flavoring agents, preservatives and antioxidants. In particular, it is desirable to mix the microbial oils with an antioxidant to prevent oxidation of the DHA or ARA. Such antioxidants would be food acceptable and could include vitamin E, carotene, BHT or other antioxidants known to those of skill in the art.

The daily dose of the compositions of the present invention to be provided to a patient will depend upon the extent of the DHA and/or ARA deficit identified by serum lipid analysis prior to the introduction of the therapy. Typically, the initial dose provided to a patient of greater than 50 pounds will be in the range of about 50 mg DHA to 5000 mg DHA per day. A preferred maintenance dose is about 500 mg DHA per day. For example, if the DHA oil to be used is 50% enriched in DHA, such a dose would correspond to the addition of about 1000 mg of oil per day.

The daily dose of ARA provided to the patient of greater than 50 pounds will be 50 mg ARA to 5000 mg per day. A preferred maintenance dose would be 500-1000 mg per day. If the ARA oil to be used is 50% enriched in ARA, such a dose would correspond to the addition of about 1000-2000 mg of ARA oil per day. Doses of a suitable combination of the DHA and ARA containing oils will be 1000 mg of DHA and 1000 mg of ARA per day.

Desirably, the patient's serum fatty acid profiles are reviewed after about four weeks of this daily therapy. Subsequent doses then can be modified in response to the observed level of plasma lipid or red blood cell DHA and ARA and in response to observed clinical responses to the therapy. Patients with peroxisomal disorders can have red blood cell levels of DHA of only 1-3 µg DHA per ml. of plasma. Normal target values range from about 10 to 30 µg of DHA per ml of plasma. Normal target values of circulating ARA range from about 75 to about 120 µg ARA per ml. of plasma. Once normalized level(s) of the circulating fatty acid(s) of interest have been achieved, the daily dose of oil(s) can be modified to maintain the circulating DHA and/or ARA at a desirable level.

As noted above, in order to treat certain neurolgoical disorders, it may be desirable to raise the level of circulating DHA and/or ARA in the blood to 4 to 5 times normal levels. The levels of circulating DHA and ARA, therefore, can be raised to about 120-150 µg/ml and about 480-600 µg/ml, respectively.

Although not wanting to be bound by any specific theory, it is the inventor's belief that the administration of DHA is effective for treating neurological disorders because of its ability to regulate calcium uptake by neuronal cells. A depolarization of the neuronal cell results in elevated levels of intracellular calcium, causing the activation of a phospholipase and resulting in the release of free DHA from the cell membrane. This free DHA acts as a calcium channel blocker, thereby limiting calcium entry into the cell. Thus, the level of DHA present in the neuronal cell membrane, and thereby available for activation-induced release of these long chain polyunsaturated fatty acids, may control intracellular calcium levels. If a deficiency of DHA exists, intracellular calcium levels rise, and the production of amyloid plaque protein is stimulated. Furthermore, high intracellular calcium stimulates the phosphorylation of the microtubule associated tau-protein, resulting in the development of neurofibrillar tangles.

Serum lipids are the most probable source of the DHA and ARA incorporated into neuronal cells, since serum lipids act as the transport or carrier system for fatty acids in general. Studies in animals and in humans have shown that high levels of DHA and ARA in the serum are correlated with high levels of DHA and ARA in the brain. Therefore, elevating the DHA and ARA concentrations in the composition of total serum lipids, by providing supplemental dietary microbial oils enriched in these components, should increase the delivery of DHA and ARA to target neuronal tissues.

The role cf ARA in neuronal function is less clear, although it too is a major component of neurological membranes. Many neurological disorders exhibit a deficiency of both DHA and ARA. The object of this invention is to supplement levels of both these components, using DHA and ARA from microbial oil to normalize both of these important fatty acids. The supplementation of DHA and ARA without any significant quantities of EPA is an important aspect of this invention, as the EPA levels in neurological tissues generally are low and supplementation with EPA will depress ARA levels, and may be contraindicated in certain instances. Therapeutic administration of the DHA oil in combination with the ARA oil may be beneficial in maintaining or establishing a ratio of ω-3 to ω-6 long chain polyunsaturated fatty acid in the body comparable to that in normal healthy individuals.

The present invention having been generally described, reference is had to the following nonlimiting specific examples.

### Example 1

A medium of one half strength artificial seawater made by combining 4.3 kg of Instant Ocean® with 230 liters of tap water was loaded into a 350 liter stirred tank fermentor. The fermentor containing the medium was sterilized and cooled to 28°C. 6.8 liters of concentrated yeast extract at a concentration of 400 grams per liter, 12.5 liters of glucose syrup at a concentration of 400 grams per liter and 30 liters of *C. cohnii* inoculum from a seed fermentor at a concentration of 10⁶ cells per ml or a biomass density of about 1.3 grams per liter were added to the medium. Agitation was set at a tip speed of 73 cm per sec and aeration was set at 1 VVM, which is equivalent to 280 liters per minute. An additional 12 liters of glucose syrup was added after about 44 hours and another 43 liters was added over the next 32 hours. Thus, 67.5 liters of glucose syrup were added in total.

To maintain the dissolved oxygen at greater than 20%, the agitation tip speed was increased to 175 cm per second at 44 hours and to 225 cm per sec at 55 hours. At 76 hours, the tip speed was decreased to 150 cm per second. The culture was grown for an additional time sufficient to convert the final charge of glucose into cellular oil, then harvested. The harvested cells were dried to about a 4% moisture content. Hexane was added to the dried biomass and stirred in a glass kettle for 2 hours at 25°C. A rotary evaporator was used to remove the hexane, producing about 700 grams of crude DHA-containing oil.

### Example 2

Sixty kg of yeast extract, 45 kg of NaCl, 12.3 kg of MgSO₄^{·}7H₂O, and 0.9 kg of CaCl₂^{·}2H₂O in 7,000 liters of water were loaded into a 15,000 liter fermentor. After this solution was sterilized, 3,000 liters of a sterilized glucose solution at a concentration of 650 kg of glucose per 3,000 liters of volume was added. The initial pH of the medium was 6.3, the temperature was 28°C, aeration was 0.5 to 1.0 VVM, the vessel back pressure was set to 0.2 bar, and the agitation tip speed was set to 120 cm per seconds before the vessel was inoculated with 300 liter of an inoculum culture of *C. cohnii* which had attained a cell density of about 60 x 10⁶ cells per ml, which is equivalent to 4 to 5 grams of dry weight of biomass per liter of culture in the inoculum tank. During the course of the fermentation, a food grade antifoam, such as Dow 1520 was added as needed and the pH was held at 6.3 using either 8 N H₂SO₄ or 4 N NaOH as needed. The dissolved oxygen level was maintained at greater than 20% of air saturation by increasing the vessel back pressure and agitation. Additional glucose additions were required at 93 hours and at 111 hours to maintain the glucose levels above 5 grams per liter. At 119 hours, the fermentor was cooled to 17°C and the fermentation broth was processed through a centrifuge. A 608 kg slurry containing 25% solids was recovered. The slurry was spray dried, producing about 150 kg dry algal powder which contained about 30 to 40 kg of oil with a DHA content of 40 to 45%.

The dry algal powder was extracted with hexane using standard vegetable oil extraction equipment and methods. Following the removal of the solvent, the crude oil was degummed by the adding water at 50°C. The degummed oil was collected by centrifugation and refined by mixing with caustic base and phosphoric acid at 55°C for one hour. The refined and degummed oil was then collected by centrifugation and gently bleached at 90°C by the addition of citric acid and bleaching clay. Filtration of the bleaching clay produced the refined, bleached oil (RB-oil) with a peroxide value of less than 5 mEq per kg. The RB-oil was deodorized by high vacuum short path distillation and the resulting deodorized RB-oil (RBD-oil) was then ready for encapsulation, tableting, or bulk shipping. The resulting oil had a peroxide value less than 1 mEq per kg, a free fatty acid content of less than 0.05%, a DHA content of 45 to 47%, and an iodine number of about 200.

### Example 3

### Preparation of Thraustochytrium aurum lipid

2.5 grams of NaCl, 5 grams of MgSO₄^{·}7H₂O, 1 gram of KCl, 0.1 grams of KH₂PO₄, 0.2 grams of CaCO₃, 0.2 grams of (NH₄)₂SO₄, 2.0 grams of sodium glutamate in 1 liter of water were loaded into a 1.7 liter stirred tank fermentor. After the tank was sterilized, a sterile solution containing 10 µgrams of thiamine-HCl, 0.1 grams of NaHCO₃, and 10 µgrams of vitamin B₁₂ was added-thiamine B₁₂ followed by the addition of 150 ml of sterile 30% glucose and 50 ml of sterile 10% yeast extract. The pH was adjusted to 6.0, the sparging was adjusted to 1.0 VVM, and agitation was adjusted to 300 rpm before inoculation with 100 ml of a 5-day old shake flask culture of *Thraustochytrium aurum* grown in the same medium. The culture was harvested after 9 days to yield about 4 grams dry weight of biomass. The DHA content of the lipid in the biomass is 10 to 15%.

### Example 4

### Preparation of Pythium insidiosum lipid

In an 80 liter (gross volume) fermentor, 51 liters of tap water, 1.2 kg glucose, 240 grams of yeast extract and 15 ml of MAZU 210S® antifoam were combined. The fermentor was sterilized at 121°C for 45 minutes. An additional 5 liters of condensate water were added during the sterilization process. The pH was adjusted to 6.2, and approximately 1 liter of inoculum at a cell density of 5 to 10 grams per liter of *Pythium insidiosum* (ATCC #28251) then was added. The agitation rate was adjusted to 125 RPM corresponding to a tip speed of 250 cm per second and the aeration rate was set at 1 SCFM (standard cubic feet per minute). At hour 24 in the operation the aeration rate was increased to 3 SCFM. At hour 28 an additional 2 liters of a 50% by weight glucose syrup were added. At hour 50 the fermentor was harvested, resulting in a yield of about 2.2 kg wet weight of biomass, which was approximately 15 grams of dry weight per liter of culture. The harvested biomass was squeezed to a high solids cake, comprising approximately 50% solids, on a suction filter before it was freeze dried. The dried biomass was ground with a mortar and pestle and extracted with 1 liter of hexane per 200 grams of dry biomass at room temperature under continuous stirring for 2 hours. The mixture then was filtered and the filtrate evaporated, yielding about 5 to 6 grams of crude oil per 100 grams of dry biomass. The biomass was reextracted with 1 liter of ethanol per 20 grams of dry biomass for 1 hour at room temperature, filtered, and the solvent evaporated, yielding an additional 22 grams of crude oil per 100 grams of dry biomass. The second fraction was predominantly phospholipids whereas the first fraction contained a mixture of phospholipids and triglycerides. The combined fractions produced an oil containing about 30 to 35% arachidonic acid and no detectable EPA.

### Example 5

### Preparation of Mortierella alpina lipid

A 7,500 liter fermentor was filled with 4,500 liters of water and charged with 225 kg dextrose, 27 kg yeast extract and 450 grams of antifoam (Dow 1520). The pH was adjusted to 5.0 and the medium was sterilized for 60 minutes at 121°C. After sterilization and cooling to 28°C, the pH was adjusted to 5.5 with NaOH, the aeration adjusted to 0.25 VVM, the back pressure set at 0.2 bar, and the agitation of the A315 impellers was set at a speed of 80 cm per second. The culture was inoculated with 180 liters of a 20 hour old seed culture of *Mortierella alpina* at 2.2 grams per liter. The pH was allowed to fluctuate until 37 hours into the run at which time it was controlled at 6.5. The agitation was increased to 110 cm per second at 26 hours during the peak oxygen demand, but it was returned to 80 cm per second at 32 hours. At 123 hours the tank was harvested using a Bock basket centrifuge fitted with a 40 micron bag. The material was then dried using a fluid bed drier and extracted with hexane as in Example 2. The fermentation yielded 17 kilograms of a crude oil with an ARA content of 45%.

### Example 6

DHA-enriched oil prepared in accordance with Example 1 or 2 was prepared for oral use by either encapsulating or tableting. Clear sealed gelatin capsules of 1 gram per capsule were prepared by conventional manufacturing methods. Banded gel caps containing one oil or a mixture of oils were prepared by allotting 250 µl of oil in the gel cap bottoms using a positive displacement manifold pipetter. With this method weight accuracy of ±3-5% was attained. The tops then were placed over the gels caps and were banded with dyed gelatin using a capsule banding machine. Alternatively, the gel cap bottoms were filled with carboxymethylcellulose and 120 µl of oil was pipetted directly onto this binder where it was adsorbed, preventing any leakage. The tops were placed over the gel caps and were banded with dyed gelatin using a capsule banding machine. Alternatively, the carboxymethylcellulose could be mixed with oil at a ratio of three parts carboxymethylcellulose to one part oil in a separate container and then pressed into tablets using a tablet press.

The procedure was repeated using ARA-enriched oil produced in accordance with Examples 4 and 5.

### Example 7

Crude microbial oils produced from microorganisms such as those described in examples 1, 2, 3, 4 and 5 were processed using conventional vegetable oil processing methods. The oil was degummed to remove phosphatides by mixing with water at 50°C, then removing the water and gum mixture by centrifugation. The oil was refined to remove the free fatty acids by mixing with caustic base followed by phosphoric acid at 55°C, then removing the water, fatty acid mixture by centrifugation. The processed microbial oil was bleached by mixing with citric acid and standard bleaching clay at 90°C before filtration to remove the spent clay and any remaining polar particles in the oil. In some cases the oil was deodorized using either a high vacuum distillation or a counter current steam stripping deodorizer resulting in the production of the final, refined, bleached and deodorized oil (RBD oil). Specifications of the oils flowing through this process typically gave a peroxide value of less than 2 mEq per kilogram and a free fatty acid level of less that 0.05%. These specifications are typical for standard vegetable oils and the microbial oils are used in this state in place of vegetable oil in the preparation of margarine, shortenings, spoonable dressings, liquid dressings, or as the oil component of other manufactured food products. The microbial oils are highly enriched in long chain polyunsaturated fatty acids, in particular DHA and ARA, and are diluted by at least one part per ten parts of a conventional oil chosen for the particular product being prepared. For incorporation into chocolate products, the oil is diluted with cocoa butter. For use as a shortening or baking product, the oil is diluted with coconut or palm oils. For use as a salad dressing, the oil is diluted with standard salad oils such as canola, soy, safflower or corn oil.

## Claims

1. Use of an oil comprising triglycerides and/or phospholipids containing docosahexanoic acid (DHA) for the preparation of a composition for the treatment of a neurological disorder associated with a DHA deficiency-associated pathology, wherein said disorder is retinitis pigmentosum, and wherein said composition is adapted for oral, nasal or topical administration.

2. Use of an oil comprising triglycerides and/or phospholipids containing arachidonic acid (ARA) for the preparation of a composition for the treatment of a neurological disorder associated with an ARA deficiency-associated pathology, said disorder selected from the group consisting of multiple sclerosis and retinitis pigmentosum.

3. Use of an oil comprising triglycerides and/or phospholipids containing docosahexanoic acid (DHA) and an oil comprising triglycerides and/or phospholipids containing arachidonic acid (ARA) for the preparation of a composition for the treatment of a neurological disorder associated with a DHA and ARA deficiency-associated pathology, said disorder selected from the group consisting of multiple sclerosis and retinitis pigmentosum, wherein said composition is adapted for oral, nasal or topical administration.

4. The use according to claim 1 or claim 3, wherein said composition is adapted for oral administration.

5. The use according to claim 1 or claim 3, wherein the oil comprising triglycerides and/or phospholipids containing docosahexanoic acid (DHA) comprises fish oil.

6. Use of an oil comprising triglycerides and/or phospholipids containing docosahexanoic acid (DHA), the oil having a ratio of DHA to EPA (eicosapentaenoic acid) of at least 10:1, for the preparation of a composition for the treatment of a neurological disorder associated with a DHA deficiency-associated pathology, said disorder selected from the group consisting of diabetic neuropathy and multiple sclerosis.

7. Use of an oil comprising triglycerides and/or phospholipids containing arachidonic acid (ARA), the oil having a ratio of ARA to EPA (eicosapentaenoic acid) of at least 10:1, for the preparation of a composition for the treatment of a neurological disorder associated with an ARA deficiency-associated pathology, wherein said disorder is diabetic neuropathy.

8. Use of an oil comprising triglycerides and/or phospholipids containing docosahexanoic acid (DHA), the oil having a ratio of DHA to EPA (eicosapentaenoic acid) of at least 10:1, and an oil comprising triglycerides and/or phospholipids containing arachidonic acid (ARA), the oil having a ratio of ARA to EPA of at least 10:1, for the preparation of a composition for the treatment of a neurological disorder associated with a DHA and ARA deficiency-associated pathology, wherein said disorder is diabetic neuropathy.

9. The use according to any one of claims 1 to 3, wherein the oil comprising triglycerides and/or phospholipids containing docosahexanoic acid (DHA) has a ratio of DHA to EPA (eicosapentaenoic acid) of at least 10:1, or wherein the oil comprising triglycerides and/or phospholipids containing arachidonic acid (ARA) has a ratio of ARA to EPA of at least 10:1.

10. The use according to any one of the preceding claims, wherein the oil comprising triglycerides and/or phospholipids containing docosahexanoic acid (DHA) and/or the oil comprising triglycerides and/or phospholipids containing arachidonic acid (ARA) comprises a microbial oil.

11. The use according to claim 10, wherein said microbial oil comprising DHA comprises at least 20% DHA.

12. The use according to claim 10, wherein said microbial oil comprising ARA comprises at least 10% ARA.

13. The use according to claim 10, wherein said microbial oil comprising DHA or ARA comprises at least 40% DHA or ARA.

14. The use according to any one of the preceding claims, wherein the composition for the treatment of a neurological disorder is capable, upon administration, of increasing blood serum levels of DHA and/or ARA in a patient or increasing levels of DHA and/or ARA in erythrocytes of a patient.

15. The use according to any one of the preceding claims, wherein the composition for the treatment of a neurological disorder is capable, upon administration, of maintaining or establishing a ratio of ω-3 to ω-6 long chain polyunsaturated fatty acids in the body comparable to that in normal healthy individuals.

16. The use according to any one of the preceding claims, wherein the DHA and/or the ARA is in the form of triglycerides.

17. The use according to any one of the preceding claims, wherein said DHA oil is obtained by cultivating at least one alga or fungus which can produce a DHA-containing oil under DHA oil-producing conditions and/or said ARA oil is obtained by cultivating at least one alga or fungus which can produce an ARA-containing oil under ARA-producing conditions, and recovering the oil(s) produced.

18. The use according to claim 17, wherein said DHA oil is obtained by cultivating a dinoflagellate alga or a Chytridiomycete or an Oomycete.

19. The use according to claim 17, wherein said alga or fungus is a species of *Crypthecodinium, Thraustochytrium, Schizochytrium, Mortierella, Saprolegnia* or *Mucor.*

20. The use according to claim 17, wherein said ARA-producing microorganism is a fungus comprising a species of *Pythium* or *Mortierella*, or an alga comprising a species of *Ochromomas, Euglena* or *Porphyridium*.

21. The use according to any one of the preceding claims, wherein said composition is in the form of a capsule, a tablet, an emulsion or a prepared food product.

## Patentansprüche

1. Verwendung eines Triglyceride und/oder Phospholipide aufweisenden Docosahexaensäure (DHA) enthaltenden Öles für die Zubereitung einer Zusammensetzung zur Behandlung einer neurologischen Störung im Zusammenhang mit einer DHA-Mangel-assoziierten Pathogenese, wobei die genannte Störung Retinitis Pigmentosa ist, und wobei die genannte Zusammensetzung für orale, nasale oder topische Verabreichung angepasst ist.

2. Verwendung eines Triglyceride und/oder Phospholipide aufweisenden Arachidonsäure (ARA) enthaltenden Öles für die Zubereitung einer Zusammensetzung zur Behandlung einer neurologischen Störung im Zusammenhang mit einer ARA-Mangel-assoziierten Pathogenese, genannte Störung ausgewählt aus der Gruppe bestehend aus multipler Sklerose und Retinitis Pigmentosa.

3. Verwendung eines Triglyceride und/oder Phospholipide aufweisenden Docosahexaensäure (DHA) enthaltenden Öles und eines Triglyceride und/oder Phospholipide aufweisenden Arachidonsäure (ARA) enthaltenden Öles für die Zubereitung einer Zusammensetzung zur Behandlung einer neurologischen Störung im Zusammenhang mit einer DHA- und ARA-Mangel-assoziierten Pathogenese, genannte Störung ausgewählt aus der Gruppe bestehend aus multipler Sklerose und Retinitis Pigmentosa, wobei die genannte Zusammensetzung für orale, nasale oder topische Verabreichung angepasst ist.

4. Die Verwendung gemäß Anspruch 1 oder Anspruch 3, wobei die genannte Zusammensetzung für orale Verabreichung angepasst ist.

5. Die Verwendung gemäß Anspruch 1 oder Anspruch 3, wobei das Triglyceride und/oder Phospholipide aufweisende Docosahexaensäure (DHA) enthaltende Öl Fischöl aufweist.

6. Verwendung eines Triglyceride und/oder Phospholipide aufweisenden Docosahexaensäure (DHA) enthaltenden Öles, wobei das Öl ein Verhältnis von DHA zu EPA (Eicosapentaensäure) von mindestens 10:1 hat, für die Zubereitung einer Zusammensetzung zur Behandlung einer neurologischen Störung im Zusammenhang mit einer DHA-Mangel-assoziierten Pathogenese, genannte Störung ausgewählt aus der Gruppe bestehend aus diabetischer Neuropathie und multipler Sklerose.

7. Verwendung eines Triglyceride und/oder Phospholipide aufweisenden Arachidonsäure (ARA) enthaltenden Öles, wobei das Öl ein Verhältnis von ARA zu EPA (Eicosapentaensäure) von mindestens 10:1 hat, für die Zubereitung einer Zusammensetzung zur Behandlung einer neurologischen Störung im Zusammenhang mit einer ARA-Mangel-assoziierten Pathogenese, wobei genannte Störung eine diabetische Neuropathie ist.

8. Verwendung eines Triglyceride und/oder Phospholipide aufweisenden Docosahexaensäure (DHA) enthaltenden Öles, wobei das Öl ein Verhältnis von DHA zu EPA (Eicosapentaensäure) von mindestens 10:1 hat, und eines Triglyceride und/oder Phospholipide aufweisenden Arachidonsäure (ARA) enthaltenden Öles, wobei das Öl ein Verhältnis von ARA zu EPA von mindestens 10:1 hat, für die Zubereitung einer Zusammensetzung zur Behandlung einer neurologischen Störung im Zusammenhang mit einer DHA- und ARA-Mangel-assoziierten Pathogenese, wobei genannte Störung eine diabetische Neuropathie ist.

9. Die Verwendung gemäß einem der Ansprüche 1 bis 3, wobei das Triglyceride und/oder Phospholipide aufweisende Docosahexaensäure (DHA) enthaltende Öl ein Verhältnis von DHA zu EPA (Eicosapentaensäure) von mindestens 10:1 hat, oder wobei das Triglyceride und/oder Phospholipide aufweisende Arachidonsäure (ARA) enthaltende Öl ein Verhältnis von ARA zu EPA von mindestens 10:1 hat.

10. Die Verwendung gemäß einem der vorhergehenden Ansprüche, wobei das Triglyceride und/oder Phospholipide aufweisende Docosahexaensäure (DHA) enthaltende Öl und/oder das Triglyceride und/oder Phospholipide aufweisende Arachidonsäure (ARA) enthaltende Öl mikrobielles Öl aufweist.

11. Die Verwendung gemäß Anspruch 10, wobei das genannte DHA enthaltende mikrobielle Öl mindestens 20% DHA enthält.

12. Die Verwendung gemäß Anspruch 10, wobei das genannte ARA enthaltende mikrobielle Öl mindestens 10% ARA enthält.

13. Die Verwendung gemäß Anspruch 10, wobei das genannte DHA oder ARA enthaltende mikrobielle Öl mindestens 40% DHA oder ARA enthält.

14. Die Verwendung gemäß einem der vorhergehenden Ansprüche, wobei die Zusammensetzung zur Behandlung einer neurologischen Störung, bei Verabreichung, in der Lage ist, die Blutserumwerte von DHA und/oder ARA bei einem Patienten zu erhöhen oder den Gehalt von DHA und/oder ARA in den Erythrozyten eines Patienten zu erhöhen.

15. Die Verwendung gemäß einem der vorhergehenden Ansprüche, wobei die Zusammensetzung zur Behandlung einer neurologischen Störung, bei Verabreichung, in der Lage ist, ein Verhältnis von ω-3 langkettigen mehrfach ungesättigten Fettsäuren zu ω-6 langkettigen mehrfach ungesättigten Fettsäuren im Körper aufrecht zu erhalten oder zu erreichen, das dem Verhältnis von normalgesunden Individuen vergleichbar ist.

16. Die Verwendung gemäß einem der vorhergehenden Ansprüche, wobei DHA und/oder ARA in der Form von Triglyceriden vorliegen.

17. Die Verwendung gemäß einem der vorhergehenden Ansprüche, wobei das genannte DHA-Öl durch Kultivierung von mindestens einer Alge oder einem Pilz, welche ein DHA enthaltendes Öl unter DHA-Öl-produzierenden Bedingungen produzieren können, gewonnen wird und/oder das genannte ARA-Öl durch Kultivierung von mindestens einer Alge oder einem Pilz, welche ein ARA enthaltendes Öl unter ARA-Öl-produzierenden Bedingungen produzieren können, gewonnen wird, und das produzierte Öl bzw. die produzierten Öle wiedergewonnen werden.

18. Die Verwendung gemäß Anspruch 17, wobei das genannte DHA-Öl durch Kultivierung einer Dinoflagellate oder einer Chytridiomycete oder einer Oomycete gewonnen wird.

19. Die Verwendung gemäß Anspruch 17, wobei die genannte Alge oder der genannte Pilz einer Species von *Crypthecodinium, Thraustochytrium*, *Schizochytrium, Mortierella*, *Saprolegnia* oder *Mucor* angehört.

20. Die Verwendung gemäß Anspruch 17, wobei der ARA-produzierende Organismus ein Pilz ist, der einer Species von *Pythium* oder *Mortierella* angehört, oder eine Alge ist, die einer Species von *Ochromonas, Euglena* oder *Porphyridium* angehört.

21. Die Verwendung gemäß einem der vorhergehenden Ansprüche, wobei die genannte Zusammensetzung in Form einer Kapsel, einer Tablette, einer Emulsion oder einer Nahrungsmittelzubereitung vorliegt.

## Revendications

1. Utilisation d'une huile comprenant des triglycérides et/ou des phospholipides contenant de l'acide docosahexanoïque (DHA) pour la préparation d'une composition destinée au traitement d'un trouble neurologique associé à une pathologie liée à une déficience en DHA, dans laquelle ledit trouble est une rétinite pigmentaire, et dans laquelle ladite composition est adaptée à l'administration orale, nasale ou topique.

2. Utilisation d'une huile comprenant des triglycérides et/ou des phospholipides contenant de l'acide arachidonique (ARA) pour la préparation d'une composition destinée au traitement d'un trouble neurologique associé à une pathologie liée à une déficience en ARA, ledit trouble étant choisi dans le groupe constitué par la sclérose en plaques et la rétinite pigmentaire.

3. Utilisation d'une huile comprenant des triglycérides et/ou des phospholipides contenant de l'acide docosahexanoïque (DHA) et d'une huile comprenant des triglycérides et/ou des phospholipides contenant de l'acide arachidonique (ARA)pour la préparation d'une composition destinée au traitement d'un trouble neurologique associé à une pathologie liée à une déficience en DHA et ARA, ledit trouble étant choisi dans le groupe constitué par la sclérose en plaques et la rétinite pigmentaire, dans laquelle ladite composition est adaptée à l'administration orale, nasale ou topique.

4. Utilisation selon la revendication 1 ou la revendication 3, dans laquelle ladite composition est adaptée à l'administration orale.

5. Utilisation selon la revendication 1 ou la revendication 3, dans laquelle l'huile comprenant des triglycérides et/ou des phospholipides contenant de l'acide docosahexanoïque (DHA) comprend une huile de poisson.

6. Utilisation d'une huile comprenant des triglycérides et/ou des phospholipides contenant de l'acide docosahexanoïque (DHA), l'huile ayant un rapport DHA/EPA (acide éicosapentaénoïque) d'au moins 10:1, pour la préparation d'une composition destinée au traitement d'un trouble neurologique associé à une pathologie liée à une déficience en DHA, ledit trouble étant choisi dans le groupe constitué par la neuropathie diabétique et la sclérose en plaques.

7. Utilisation d'une huile comprenant des triglycérides et/ou des phospholipides contenant de l'acide arachidonique (ARA), l'huile ayant un rapport ARA/EPA (acide éicosapentaénoïque) d'au moins 10:1, pour la préparation d'une composition destinée au traitement d'un trouble neurologique associé à une pathologie liée à une déficience en ARA, dans laquelle ledit trouble est une neuropathie diabétique.

8. Utilisation d'une huile comprenant des triglycérides et/ou des phospholipides contenant de l'acide docosahexanoïque (DHA), l'huile ayant un rapport DHA/EPA (acide éicosapentaénoïque) d'au moins 10:1, et d'une huile comprenant des triglycérides et/ou des phospholipides contenant de l'acide arachidonique (ARA), l'huile ayant un rapport ARA/EPA d'au moins 10:1, pour la préparation d'une composition destinée au traitement d'un trouble neurologique associé à une pathologie liée à une déficience en DHA et ARA, dans laquelle ledit trouble est une neuropathie diabétique.

9. Utilisation selon l'une quelconque des revendications 1 à 3, dans laquelle l'huile comprenant des triglycérides et/ou des phospholipides contenant de l'acide docosahexanoïque (DHA) a un rapport DHA/EPA (acide éicosapentaénoïque) d'au moins 10:1, ou dans laquelle l'huile comprenant des triglycérides et/ou des phospholipides contenant de l'acide arachidonique (ARA) a un rapport ARA/EPA d'au moins 10:1.

10. Utilisation selon l'une quelconque des revendications précédentes, dans laquelle l'huile comprenant des triglycérides et/ou des phospholipides contenant de l'acide docosahexanoïque (DHA) et/ou l'huile comprenant des triglycérides et/ou des phospholipides contenant de l'acide arachidonique (ARA) comprennent une huile microbienne.

11. Utilisation selon la revendication 10, dans laquelle ladite huile microbienne comprenant du DHA comprend au moins 20% de DHA.

12. Utilisation selon la revendication 10, dans laquelle ladite huile microbienne comprenant de l'ARA comprend au moins 10% d'ARA.

13. Utilisation selon la revendication 10, dans laquelle ladite huile microbienne comprenant du DHA ou de l'ARA comprend au moins 40% de DHA ou d'ARA.

14. Utilisation selon l'une quelconque des revendications précédentes, dans laquelle la composition destinée au traitement d'un trouble neurologique est capable, après administration, d'augmenter les taux de DHA et/ou d'ARA dans le sérum sanguin d'un patient ou d'augmenter les taux de DHA et/ou d'ARA dans les érythrocytes d'un patient.

15. Utilisation selon l'une quelconque des revendications précédentes, dans laquelle la composition destinée au traitement d'un trouble neurologique est capable, après administration, de maintenir ou d'établir dans l'organisme un rapport des acides gras polyinsaturés à chaîne longue ω-3 aux acides gras polyinsaturés à chaîne longue ω-6 qui est comparable à celui des individus normaux sains.

16. Utilisation selon l'une quelconque des revendications précédentes, dans laquelle le DHA et/ou l'ARA sont sous forme de triglycérides.

17. Utilisation selon l'une quelconque des revendications précédentes, dans laquelle ladite huile de DHA s'obtient en cultivant au moins une algue ou un champignon qui peut produire une huile contenant du DHA dans les conditions de production d'huile de DHA et/ou ladite huile d'ARA s'obtient en cultivant au moins une algue ou un champignon qui peut produire une huile contenant de l'ARA dans les conditions de production d'ARA, et en récupérant la ou les huile(s) produite(s).

18. Utilisation selon la revendication 17, dans laquelle ladite huile de DHA s'obtient en cultivant une algue du groupe des Dinoflagellés ou un Chytridiomycète ou un Oomycète.

19. Utilisation selon la revendication 17, dans laquelle ladite algue ou ledit champignon est une espèce de *Crypthecodinium, Thraustochytrium, Schizochytrium, Mortierella, Saprolegnia* ou *Mucor*.

20. Utilisation selon la revendication 17, dans laquelle ledit micro-organisme producteur d'ARA est un champignon comprenant une espèce de *Pythium* ou de *Mortierella*, ou une algue comprenant une espèce d'*Ochromonas*, d'*Euglena* ou de *Phorphyridium*.

21. Utilisation selon l'une quelconque des revendications précédentes, dans laquelle ladite composition se présente sous forme d'une capsule, d'un comprimé, d'une émulsion ou d'un produit alimentaire préparé.
